# EUROPEAN PATENT APPLICATION

(11) **EP 4 600 965 A1**
(43) Date of publication of application: **13.08.2025**
(21) Application number: 24156528.2
(22) Date of filing: 08.02.2024
(51) Int. Cl.: G16B 30/10

(54) **METHOD FOR IDENTIFYING OFF-TARGET PROTEINS**

(71) Applicant: BenevolentAI Technology Limited, London Greater London W1T 5HD (GB)
(72) Inventor: Potterton, Andrew, London, W1T 5HD (GB); Meyers, Joshua, London, W1T 5HD (GB); Angonese do Canto, Bibiana, London, W1T 5HD (GB)
(74) Representative: Gill Jennings & Every LLP

(57) **Abstract**

A computer-implemented method for identifying off-target proteins is disclosed. The method comprises: receiving an indication of a drug target, wherein the drug target is a first protein comprising residues of interest for targeting; receiving data indicative of a whole protein sequence corresponding to the first protein; comparing the whole protein sequence of the first protein against a protein sequence database to identify whole protein sequences of other proteins having a threshold level of sequence resemblance to the whole protein sequence of the first protein; performing multiple sequence alignment on the whole protein sequences of the other proteins with respect to the whole protein sequence of the first protein; identifying residues within each of the aligned whole protein sequences of the other proteins which positionally correspond with the residues of interest in the whole protein sequence of the first protein; determining a measure of similarity between the first protein and each respective other protein; and identifying one or more of the other proteins as off-target proteins with respect to the drug target based on the measures of similarity.

## Description

### FIELD OF THE INVENTION

The present invention relates to a computer-implemented method for identifying off-target proteins.

### BACKGROUND

A drug target is a biological entity in the body, such as a protein, that is intrinsically related to a particular disease process and may be addressed by a drug to produce a desired therapeutic effect with respect to the particular disease. To identify a drug which is both effective and safe, it is vital to assess and understand the selectivity of drugs with respect to potential drug targets. For example, if a drug has a low selectivity with respect to a drug target, the drug is likely to interact with various other biological entities and produce off-target side effects which may lead to safety issues or low dose tolerability.

Biological entities, other than the (primary) drug target, which are modulated by a drug for the (primary) drug target are known as off-targets. In other words, off-targets are biological entities that are associated with unintended therapeutic modalities for a particular drug, i.e. the drug will simultaneously interact with the drug target and the off-targets. In the drug discovery process, the identification of off-targets therefore plays a fundamental role in the assessment of the selectivity of drugs with respect to drug targets.

Previous approaches to identify off-targets with respect to a primary target protein have been based on a comparison and assessment of the similarity between pockets in the primary target protein to (known) pockets in other proteins in the human proteome (~ 20,000 proteins). Pockets are cavities on the protein surface that have the potential to allow other entities, including small molecules (i.e. ligands), peptides and other proteins to bind to the protein. The single amino acid monomers which define or surround a pocket are known as pocket residues. The assessment of similarity is performed by comparing pocket sequences (i.e. the segment of the protein sequence starting from the first pocket residue to the last pocket residue) in the primary target protein to each pocket sequence in each protein in the human proteome. However, the process of performing pocket-to-pocket comparisons across the human proteome involves a high computation cost. In addition, this previous approach requires knowledge of the pocket sequences in each protein otherwise comparisons cannot be performed, i.e. high quality pocket detection across proteins in the human proteome is essential for reliable off-target identification.

An object of the present invention is therefore to provide an improved method for identifying off-target proteins. In particular it is desirable to provide an off-target protein identification method with increased computational efficiency compared to previous methods.

### SUMMARY OF INVENTION

According to a first aspect of the invention, there is provided a computer-implemented method for identifying off-target proteins, comprising: receiving an indication of a drug target, wherein the drug target is a first protein comprising residues of interest for targeting; receiving data indicative of a whole protein sequence corresponding to the first protein, wherein the data indicative of the whole protein sequence includes annotations of the residues of interest; comparing the whole protein sequence of the first protein against a protein sequence database to identify whole protein sequences of other proteins having a threshold level of sequence resemblance to the whole protein sequence of the first protein; performing multiple sequence alignment on the whole protein sequences of the other proteins with respect to the whole protein sequence of the first protein; identifying residues within each of the aligned whole protein sequences of the other proteins which positionally correspond with the residues of interest in the whole protein sequence of the first protein; determining a measure of similarity between the first protein and each respective other protein based on the degree of matching between the residues of interest in the whole protein sequence of the first protein to the identified corresponding residues in the whole protein sequence of the respective other protein; and identifying one or more of the other proteins as off-target proteins with respect to the drug target based on the measures of similarity.

In this way, off-target proteins with respect to a drug target (e.g. for a particular drug) can be identified with high computational efficiency. This is achieved in view of the following considerations.

Firstly, it has been found that proteins with similar overall protein sequences will likely contain similar groupings of residues. For example, if the first protein contains residues of interest which define a pocket, then it is likely that other proteins that share a similar overall protein sequence will also contain similar residues which define similar pockets. Hence, by performing an initial comparison between the overall protein sequence of the first protein (i.e. the query protein) with the overall protein sequences of all other proteins in a protein sequence database and eliminating all other proteins which do not sufficiently resemble the overall protein sequence of the first protein, the method is able to significantly narrow the search space for off-targets. For example, when the protein sequence database is a protein sequence database of the human proteome, the method may reduce the search space for subsequent steps of the method from approximately 20,000 proteins to fewer than 400 proteins.

Secondly, again using the specific example of pocket residues, rather than performing direct pocket-to-pocket comparisons which requires prior knowledge of the pocket sequences in the first protein and all other proteins with which a comparison is performed, the method operates by comparing the residues of interest in the first protein to residues in other proteins which positionally correspond to the residues of interest. In particular, multiple sequence alignment is performed on the other proteins with respect to the first protein, and a comparison is performed between each residue of interest in the first protein to the respective residue in each other protein which is located in an equivalent position in the aligned protein sequence. In other words, in the context of pocket residues, the method of the present invention compares the pocket of the query protein to the overall protein sequence of each other protein (i.e. pocket-to-protein comparisons). The present invention therefore provides a significant improvement in computational efficiency compared to previous approaches which relied on the performance of pocket-to-pocket comparisons, and thereby involved a much higher computation complexity of (M.N)², where M is the number of proteins and N is the number of pockets.

In addition to the benefits in terms of competition efficiency, the present invention also does not require any knowledge of pocket locations in the other proteins, so the method is not limited by the overall quality of pocket detection (or the detection of other types of residues). Furthermore, the present invention is advantageous over other previous methods which take into account the 3D shape of the pocket when performing comparisons. For these 3D methods, each comparison is specific to a confirmation of a pocket, meaning that the search space of the query is large and it is not possible to identify off-targets which do not have a known 3D structure. The present invention overcomes these issues by only utilising protein sequences, and not protein structures.

It will be appreciated that the residues of interest for targeting may be residues which are targetable by a particular drug, e.g. the residues of interest may correspond to a targetable protein binding pocket. Alternatively, the residues of interest for targeting may be residues which are of general interest when studying the targeting of the first protein, e.g. the residues of interest may be surface lysines, with the conservation of surface lysines being important for target degradation (i.e. PROTAC) approaches.

Preferably, wherein the residues of interest are protein binding pocket residues of interest. That is, the first protein comprises a binding pocket defined or surrounded by the residues of interest. Alternatively, the residues of interest may be surface lysines.

The skilled person will understand that the measure of similarity between the first protein and each respective other protein is determined based on the degree of matching between the residues of interest in the whole protein sequence of the first protein to the identified corresponding residues in the whole protein sequence of the respective other protein only. That is, the method does not perform any further comparisons between other portions of the protein sequences in the first protein and the other protein, and determining the measure of similarity only requires a comparison between the residues of interest in the first protein and the positionally correspondent residues in the other proteins.

Preferably, wherein each measure of similarity is determined based on an evaluation of the expression n/m, wherein n is the number of exact matches between the residues of interest in the whole protein sequence of the first protein to the identified corresponding residues in the whole protein sequence of the respective other protein, and wherein m is the number of residues of interest. The skilled person will understand that an exact match means that the residue of interest in the first protein is identical to the corresponding residue in the respective other protein. In other words, each measure of similarity is determined based on a percentage of identity between the residues of interest in the first protein to the corresponding residues in the aligned protein sequence of the respective other protein. This may be referred to as an identity scoring approach.

Preferably, wherein each measure of similarity is determined based on an evaluation of the expression n/m, wherein n is the number of similar matches between the residues of interest in the whole protein sequence of the first protein to the identified corresponding residues in the whole protein sequence of the respective other protein, and wherein m is the number of residues of interest. In other words, each measure of similarity is determined based on a percentage of similarity between the residues of interest in the first protein to the corresponding residues in the aligned protein sequence of the respective other protein. This may be referred to as a similarity scoring approach. This approach may overcome issues associated with the identity scoring approach, e.g. using the identity scoring approach may provide a false impression of difference for functionally similar residues.

Preferably, wherein similar matching residues are residues which are determined to fall within a same predefined amino acid property group. In other words, if the residue of interest and a respective residue are determined to be within the same property group (e.g. the residues are both small hydrophobic residues), the residue of interest and the respective residue are determined to be a similar match. Alternatively, similar matches between residues may be determined based on an evolutionary similarity matrix.

Preferably, wherein the data indicative of the whole protein sequence corresponding to the first protein comprises a string of uppercase and lowercase characters, and wherein the lowercase characters are the annotations of the residues of interest. In this way, an efficient method of encoding information regarding the location of residues (e.g. which residues are in a pocket) is provided. This information is retained following multiple sequence alignment of the protein sequences.

Preferably, wherein a profile hidden Markov model based algorithm is used to identify the whole protein sequences of the other proteins in the protein sequence database having the threshold level of sequence resemblance to the whole protein sequence of the first protein. For example, a profile may be built from the protein sequence of the first protein, using a simple position-independent scoring system (e.g. BLOSUM scores converted to probabilities, plus a gap-open and gap-extend probability). This profile can be then searched against other profiles in the protein sequence database and whole protein sequences of the other proteins in the protein sequence database having a threshold level of sequence resemblance to the whole protein sequence of the first protein are returned. In one example, the Phmmer search tool may be used to identify the whole protein sequences of the other proteins in the protein sequence database having the threshold level of sequence resemblance to the whole protein sequence of the first protein.

Preferably, wherein progressive and/or iterative refinement algorithm(s) are used to perform the multiple sequence alignment on the whole protein sequences of the other proteins with respect to the whole protein sequence of the first protein. Multiple sequence alignment (MSA) tools and programs are well-known in the art. In one example, the MAAFT sequence alignment tool (e.g. MAFFT L-INS-i) may be used to perform the multiple sequence alignment on the whole protein sequences of the other proteins with respect to the whole protein sequence of the first protein.

Preferably, wherein identifying the one or more of the other proteins as off-target proteins based on the measures of similarity comprises: identifying the one or more of the other proteins as off-target proteins with respect the drug target in response to determining that each measure of similarity for the one or more of the other proteins exceeds a threshold measure of similarity.

Preferably, wherein the drug target is targetable by a first drug. More preferably, wherein the drug target is associated with a first disease. More preferably, wherein the method further comprises selecting the first drug as a drug for the disease based on the identified off-target proteins.

Preferably, wherein the method further comprises identifying the first protein as a drug target based on the one or more off-targets.

According to a second aspect of the invention, there is provided a computer-implemented method for identifying a drug target, comprising: receiving an indication of a first protein comprising residues of interest for targeting; receiving data indicative of a whole protein sequence corresponding to the first protein, wherein the data indicative of the whole protein sequence includes annotations of the residues of interest; comparing the whole protein sequence of the first protein against a protein sequence database to identify whole protein sequences of other proteins having a threshold level of sequence resemblance to the whole protein sequence of the first protein; performing multiple sequence alignment on the whole protein sequences of the other proteins with respect to the whole protein sequence of the first protein; identifying residues within each of the aligned whole protein sequences of the other proteins which positionally correspond with the residues of interest in the whole protein sequence of the first protein; determining a measure of similarity between the first protein and each respective other protein based on the degree of matching between the residues of interest in the whole protein sequence of the first protein to the identified corresponding residues in the whole protein sequence of the respective other protein; and selecting the first protein as a drug target based on the measures of similarity.

Preferably, wherein selecting the first protein as the drug target based on the measures of similarity comprises: selecting the first protein as the drug target in response to determining that all the measures of similarity are each below a threshold measure of similarity.

Preferably, the method further comprises: receiving an indication of a second protein comprising residues of interest for targeting; receiving data indicative of a whole protein sequence corresponding to the second protein, wherein the data indicative of the whole protein sequence includes annotations of the residues of interest; comparing the whole protein sequence of the second protein against a protein sequence database to identify whole protein sequences of other proteins having a threshold level of sequence resemblance to the whole protein sequence of the second protein; performing multiple sequence alignment on the whole protein sequences of the other proteins with respect to the whole protein sequence of the second protein; identifying residues within each of the aligned whole protein sequences of the other proteins which positionally correspond with the residues of interest in the whole protein sequence of the second protein; determining a measure of similarity between the second protein and each respective other protein based on the degree of matching between the residues of interest in the whole protein sequence of the second protein to the identified corresponding residues in the whole protein sequence of the respective other protein; and selecting the first protein as a drug target based on the measures of similarity between the first protein and the other proteins and based on the measures of similarity between the second protein and the other proteins.

According to a third aspect of the invention, there is provided a method for selecting a protein pocket for targeting, comprising: receiving an indication of a first protein comprising a first pocket of interest for targeting and a second pocket of interest for targeting; receiving data indicative of a whole protein sequence corresponding to the first protein, wherein the data indicative of the whole protein sequence includes annotations of first and second residues corresponding to the first and second pockets respectively; comparing the whole protein sequence of the first protein against a protein sequence database to identify whole protein sequences of other proteins having a threshold level of sequence resemblance to the whole protein sequence of the first protein; performing multiple sequence alignment on the whole protein sequences of the other proteins with respect to the whole protein sequence of the first protein; identifying residues within each of the aligned whole protein sequences of the other proteins which positionally correspond with the first residues in the whole protein sequence of the first protein; determining a first measure of similarity between the first protein and each respective other protein based on the degree of matching between the first residues in the whole protein sequence of the first protein to the identified corresponding residues in the whole protein sequence of the respective other protein; identifying residues within each of the aligned whole protein sequences of the other proteins which positionally correspond with the second residues in the whole protein sequence of the first protein; determining a second measure of similarity between the first protein and each respective other protein based on the degree of matching between the second residues in the whole protein sequence of the first protein to the identified corresponding residues in the whole protein sequence of the respective other protein; and selecting the first pocket as a drug target based on the first and second measures of similarity.

It will be appreciated that the preferable clauses of the first aspect apply equally to the second and third aspects.

According to a fourth aspect of the invention, there is provided a computer-readable medium comprising data or instruction code, which when executed on a processor, causes the processor to implement the computer-implemented method of the first, second or third aspects.

According to a fifth aspect of the invention, there is provided a computer program product comprising instructions which, when the program is executed by a computer, cause the computer to carry out the method of the first, second or third aspects.

### BRIEF DESCRIPTION OF DRAWINGS

Embodiments of the invention are now described, by way of example, with reference to the drawings, in which:
Figure 1 is a flowchart showing method steps for identifying off-target proteins;
Figure 2 is a sequence of diagrams illustrating an exemplary alignment and scoring of protein sequences; and
Figure 3 is a block diagram of system components of a computing system in an embodiment of the invention.

### DETAILED DESCRIPTION

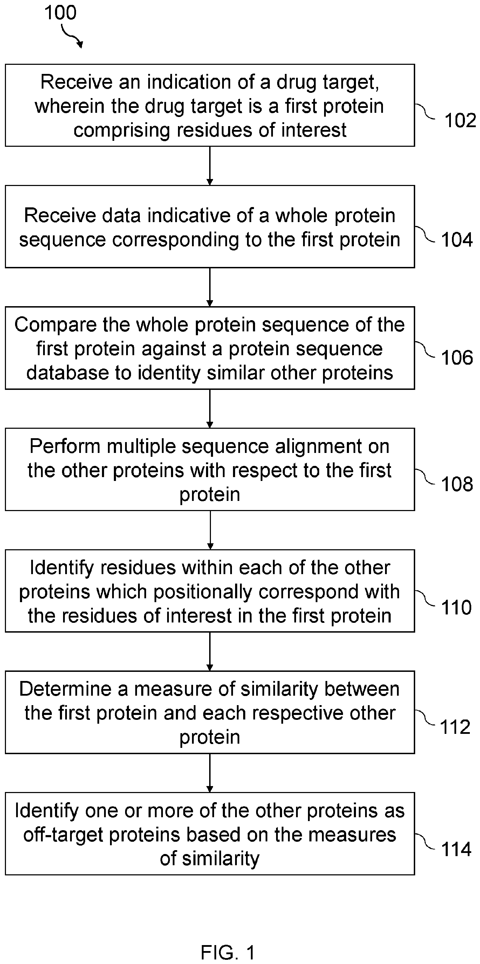

Figure 1 illustrates a method 100 of identifying off-target proteins. It will be understood that off-target proteins are proteins that are at risk of being fortuitously and simultaneously targeted by a drug for a primary target protein. That is, off-target proteins are proteins, other than the primary target protein, which will interact with a drug targeted at the primary target protein.

The method 100 begins at step 102 wherein an indication of a drug target is received. The drug target is a first protein which comprises residues of interest for targeting. In the context of the present invention, the skilled person will understand the term "residue" to refer to a single amino acid unit of a protein. For ease of understanding, the following description of method 100 will be based on a specific embodiment in which the residues of interest are binding pocket residues, i.e. the first protein comprises a targetable binding pocket. A binding pocket is a cavity on the protein surface that possesses suitable properties to allow other entities, such as ligands, peptides and other proteins, to bind to the protein. Binding pocket residues of interest will therefore be understood to refer to the residues of the first protein which form the binding pocket, e.g. the residues which define or surround the cavity. However, it will be appreciated that the method of the present invention is not limited to the residues of interest being binding pocket residues. For example, the residues of interest for targeting may instead be surface lysines, with the conservation of surface lysines being important for target degradation (i.e. PROTAC) approaches.

At step 104, data indicative of a whole protein sequence corresponding to the first protein is received. A whole protein sequence will be understood to refer to the sequence of amino acids from the amino-terminal to the carboxyl-terminal of a protein, and will usually be notated as a string of letters or characters, e.g. with each letter corresponding to a particular amino acid. The data indicative of the whole protein sequence may be, for example, a file containing a sequence in a standard format such as FASTA or UniProtKB/Swiss-Prot format. This may be retrieved from a protein sequence database (e.g. from data storage unit 306) in response to receiving the indication of the drug target comprising the first protein, or may be received simultaneously with or subsequent to the indication of the drug target comprising the first protein.

The whole protein sequence of the first protein includes sequence annotations which identify locations of the residues of interest within the first protein. For example, the residues of interest may be annotated using lowercase letters in a protein sequence string of otherwise uppercase letters.

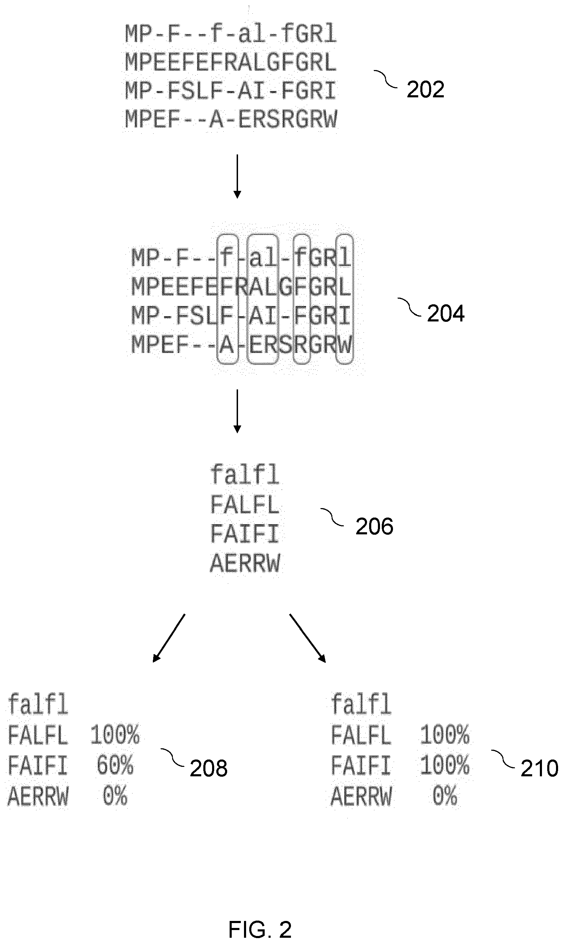

Figure 2 is a series of diagrams 202 - 210 illustrating a number of exemplary protein sequences which are received, aligned and scored in accordance with the method of the present invention. An exemplary annotated protein sequence of the first protein is illustrated in the upper row of diagram 202, e.g. amino acids f (phenylalanine), a (alanine), I (leucine), f (phenylalanine), and I (leucine) are the residues of interest which correspond to a binding pocket that has been identified for targeting in the first protein. It will be appreciated that this sequence (and the other sequences illustrated in Figure 2) have been shortened for ease of illustration.

At step 106, the whole protein sequence of the first protein is searched against a protein sequence database to identify other similar or homologous protein sequences. The protein sequence database may be a human protein sequence database (i.e. a database for the human proteome), such as UniProt.

In particular, the search is performed to identify whole proteins sequences of other proteins which have a threshold level of sequence resemblance to the first protein. The threshold level of sequence resemblance may be correspond to a statistically significant similarity that reflects common ancestry or is indicative of a shared evolutionary history. To this end, a searching tool (e.g. Phmmer) may be utilised which uses the whole protein sequence of the first protein as a query sequence, and identifies other similar proteins in a protein sequence database using profile hidden Markov Models. In particular, a profile may be built from the whole protein sequence of the first protein using a simple position-independent scoring system, e.g. BLOSUM scores converted to probabilities, plus a gap-open and gap-extend probability. This profile can then be searched against other profiles in the protein sequence database, and whole protein sequences of the other proteins in the protein sequence database having a threshold level of sequence resemblance to the whole protein sequence of the first protein are returned.

It has been found that other proteins which share a similar overall protein sequence to first protein will also contain similar residues which define similar pockets. Hence, by comparing the whole protein sequence of the first protein with the whole protein sequences of all other proteins in a protein sequence database and eliminating all other proteins which do not sufficiently resemble the overall protein sequence of the first protein, the method 100 is able to significantly narrow the search space for off-targets. For example, when the protein sequence database is a protein sequence database of the human proteome, the method may reduce the search space for subsequent steps of the method from approximately 20,000 proteins to fewer than 400 proteins.

At step 108, multiple sequence alignment (MSA) is performed on the whole protein sequences of the other proteins with respect to the whole protein sequence of the first protein. Multiple sequence alignment is an algorithmic process of aligning protein sequences to reflect their evolutionary, functional or structural relationship. This is achieved by inserting gaps of differing length within each sequence, allowing homologous positions to be aligned with each other. That is, multiple sequence alignment results in corresponding residues across sequences generally being arranged in the same column. An example of aligned protein sequences resulting from multiple sequence alignment is seen in diagram 202 of Figure 2. The second, third and fourth rows of diagram 202 each illustrate exemplary protein sequences of other proteins (henceforth referred to as the second, third, and fourth proteins respectively) which have been identified as having a threshold level of sequence resemblance to the whole protein sequence of the first protein, and have undergone multiple sequence alignment with respect to the whole protein sequence of the first protein (i.e. the protein sequence illustrated in the first row of diagram 202). The inserted gaps in the aligned protein sequences of the second, third, and fourth protein sequences are represented as hyphens.

The skilled person will be aware of various multiple sequence alignment algorithms which can be used for performance of method step 104. For example, progressive and/or iterative refinement algorithm(s), such as the MAAFT sequence alignment tool (e.g. MAFFT L-INS-i), may be used to perform multiple sequence alignment on the whole protein sequences of the other proteins with respect to the whole protein sequence of the first protein.

At step 110, following multiple sequence alignment, the aligned whole protein sequences of the other proteins are compared to the whole protein sequence of the first protein to identify residues in the aligned whole protein sequences of the other proteins which positionally correspond to the residues of interest in the protein sequence of the first protein. In other words, for each aligned protein sequence of the other proteins, residues which are arranged in the same column as one of the residues of interest are identified.

This is exemplified in diagrams 204 and 206 of Figure 2. Diagram 204 illustrates the identification of residues within the other proteins (i.e. the second, third and fourth proteins) which are aligned with the binding pocket residues of interest in the first protein. In particular, diagram 204 illustrates a plurality of columns which respectively encompass residues of interest in the first protein and identified equivalent residues in the second, third and fourth proteins. For example, for the second protein, residues F (phenylalanine), A (alanine), L (leucine), F (phenylalanine), and L (leucine) are identified as positionally corresponding to the residues of interest f (phenylalanine), a (alanine), I (leucine), f (phenylalanine), and I (leucine) respectively. For the third protein, residues F (phenylalanine), A (alanine), I (isoleucine), F (phenylalanine), and I (isoleucine) are identified as positionally corresponding to the residues of interest f (phenylalanine), a (alanine), I (leucine), f (phenylalanine), and I (leucine) respectively. For the fourth protein, residues A (alanine), E (glutamic acid), R (arginine), R (arginine), and W (tryptophan) are identified as positionally corresponding to the residues of interest f (phenylalanine), a (alanine), l (leucine), f (phenylalanine), and l (leucine) respectively.

Diagram 206 is a simplified version of diagram 204 in which only the residues are interest and identified positionally correspondent residues are shown.

At step 112, measures of similarity between the first protein and each of the other proteins are calculated. For example, a measure of similarity between the first protein and a second protein of the other proteins is calculated, a measure of similarity between the first protein and a third protein of the other proteins is calculated, and so forth. This is achieved by assessing the degree of matching between the residues of interest in the first protein to the positionally correspondent residues in the respective other protein.

The measures of similarity may be determined using a number of different approaches. According to a first approach, the measure of similarity may be determined based on an assessment of the percentage of identity between the residues of interest in the first protein and the positionally correspondent residues in the respective other protein, i.e. based on the number of identical residues between the residues of interest and the positionally correspondent residues in the respective other protein. The percentage of identity may be calculated based on the expression *n*/*m*, wherein *n* is the number of identical matches between the residues of interest in the whole protein sequence of the first protein to the identified corresponding residues in the whole protein sequence of the respective other protein, and wherein *m* is the number of residues of interest in the first protein. Diagram 208 in Figure 2 illustrates a calculation of the percentage of identity for each of the other protein sequences. For the second protein, the number of identical residues is 5 and the number of residues of interest is 5, which results in a percentage of identity of 1 or 100%. For the third protein, the number of identical residues is 3 and the number of residues of interest is 5, which results in a percentage of identity of 0.6 or 60%. For the fourth protein, the number of identical residues is 0 and the number of residues of interest is 5, which results in a percentage of identity of 0 or 0%. It will be understood that the measure of similarity may correspond to (e.g. consist of) or be derived from the calculated percentage of identity.

According to a second approach, the measure of similarity may be determined based on an assessment of the percentage of similarity between the residues of interest in the first protein and the positionally correspondent residues in the respective other protein, i.e. based on the number of similar residues between the residues of interest and the positionally correspondent residues in the respective other protein. Similar residues may amino acid residues having similar physicochemical properties. For example, similar residues may be defined as residues which fall within a same predefined amino acid property group. Examples of amino acid property groups include:

| **Group** | **Amino acids** |
|---|---|
| Small hydrophobic | A, G, I, L, V |
| Large hydrophobic | F, W, Y |
| Contains sulphur | C, M |
| Polar | S, T |
| Positively charged, basic | K, R, H |
| Negatively charged, polar | D, E, Q, N |
| Proline | P |

Alternatively, similar residues may be determined based on an evolutionary similarity matrix.

The percentage of similarity may be calculated based on the expression *n*/*m*, wherein *n* is the number of similar matches between the residues of interest in the whole protein sequence of the first protein to the identified corresponding residues in the whole protein sequence of the respective other protein, and wherein *m* is the number of residues of interest. Diagram 210 in Figure 2 illustrates a calculation of the percentage of similarity for each of the other protein sequences. For the second protein, the number of similar residues is 5 and the number of residues of interest is 5, which results in a percentage of identity of 1 or 100%. For the third protein, the number of identical residues is 5 (e.g. leucine and isoleucine are determined to fall within the same amino acid property group) and the number of residues of interest is 5, which results in a percentage of identity of 0.6 or 60%. For the fourth protein, the number of identical residues is 0 (e.g. none of the residues are determined to fall within the same amino acid property group) and the number of residues of interest is 5, which results in a percentage of identity of 0 or 0%. It will be understood that the measure of similarity may correspond to (e.g. consist of) or be derived from the calculated percentage of similarity.

As the measures of similarity are calculated based on pocket-to-protein comparisons, the method 100 provides a significant improvement in computational efficiency compared to previous approaches for identifying similar proteins which rely on pocket-to-pocket comparisons involving a computation complexity of (M.N)² where M is the number of proteins and N is the number of pockets. In addition, as the method 100 does not require any knowledge of pocket locations in the other proteins, the identification of similar proteins using method 100 is not limited by the quality of pocket detection in the other proteins.

At step 114, one or more off-targets proteins are identified based on the calculated measures of similarity. In particular, it is determined whether each of the other proteins is an off-target with respect to the first protein based on their respective calculated measures of similarity.

In this regard, it will be appreciated that a high measure of similarity between the first protein and another particular protein indicates that the particular protein will also interact with a drug targeted at the first protein, i.e. that the particular protein is an off-target with respect to the first protein. Hence, in one example, one or more of the other proteins may be identified as off-target proteins with respect to the first protein in response to determining that each measure of similarity for the one or other proteins exceeds a threshold measure of similarity, e.g. a second protein of the other proteins is determined to be an off-target if its measure of similarity exceeds the threshold measure of similarity, a third protein of the other proteins is determined to be an off-target if its measure of similarity exceeds the threshold measure of similarity, etc.

In a first specific example, referring to diagram 208 of Figure 2, if the threshold measure of similarity is 80%, then the second protein will be determined to be an off-target with respect to the first protein. In a second specific example, referring to diagram 210 of Figure 2, if the threshold measure of similarity is 95%, then the second and third proteins will be determined to be off-targets with respect to the first protein.

Although the above description has focused on the identification of off-target proteins, it will be appreciated that the method 100 may be adapted to provide alternative outputs. For example, the method 100 may be modified to instead provide a method for identifying drug targets. In this case, the method may be modified such that an indication of a first protein comprising residues of interest for targeting is received at a modified method step 102, and the first protein is selected as a drug target based on the measures of similarity at a modified method step 114. For example, the first protein may be selected as the drug target in response to determining that all the measures of similarity are each below a threshold measure of similarity. The method may also further comprise receiving an indication of a second protein comprising residues of interest for targeting, performing method steps 104 to 112 with respect to the second protein, and selecting the first protein as a drug target (and not selecting the second protein as a drug target) based on the measures of similarity between the first protein and the other proteins and based on the measures of similarity between the second protein and the other proteins. In other words, the first protein is determined to be a more suitable drug target than the second protein based on the measures of similarity.

It will also be appreciated that the final method step 114 may be omitted, and the method 100 may simply output the measures of similarity in combination with an indication of the identified positionally correspondent residues for each of the other proteins. This data may be output in the form of a FASTA file. Progressability assessments for the first protein as a target can then be performed based on the likelihood that selectivity can be achieved, e.g. based on the measures of similarity.

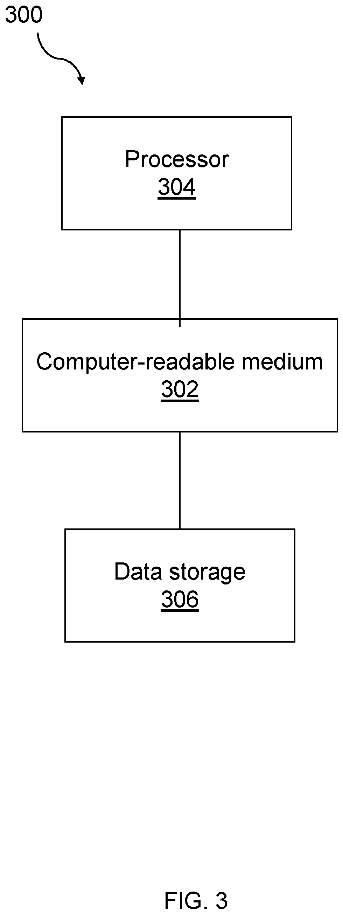

Figure 3 is a block diagram of system components of a computing system 300. The computing system 300 comprises a computer readable medium 302 provided in electronic communication (e.g. wired or wireless) with a processor 304 and a data storage unit 306. The computer readable medium 302 may comprise memory storing a computer program having computer-executable instructions for performing the various aspects of the method disclosed above. The data storage unit 306 may be remote from the computing system, and may contain data such as protein sequence information and pocket location for use in steps 104 and 106.

## Claims

1. A computer-implemented method for identifying off-target proteins, comprising:
receiving an indication of a drug target, wherein the drug target is a first protein comprising residues of interest for targeting;
receiving data indicative of a whole protein sequence corresponding to the first protein, wherein the data indicative of the whole protein sequence includes annotations of the residues of interest;
comparing the whole protein sequence of the first protein against a protein sequence database to identify whole protein sequences of other proteins having a threshold level of sequence resemblance to the whole protein sequence of the first protein;
performing multiple sequence alignment on the whole protein sequences of the other proteins with respect to the whole protein sequence of the first protein;
identifying residues within each of the aligned whole protein sequences of the other proteins which positionally correspond with the residues of interest in the whole protein sequence of the first protein;
determining a measure of similarity between the first protein and each respective other protein based on the degree of matching between the residues of interest in the whole protein sequence of the first protein to the identified corresponding residues in the whole protein sequence of the respective other protein; and
identifying one or more of the other proteins as off-target proteins with respect to the drug target based on the measures of similarity.

2. The method of claim 1, wherein the residues of interest are protein binding pocket residues.

3. The method of claim 1 or claim 2, wherein each measure of similarity is determined based on an evaluation of the expression *n*/*m*, wherein *n* is the number of exact matches between the residues of interest in the whole protein sequence of the first protein to the identified corresponding residues in the whole protein sequence of the respective other protein, and wherein *m* is the number of residues of interest.

4. The method of claim 1 or claim 2, wherein each measure of similarity is determined based on an evaluation of the expression *n*/*m,* wherein *n* is the number of similar matches between the residues of interest in the whole protein sequence of the first protein to the identified corresponding residues in the whole protein sequence of the respective other protein, and wherein *m* is the number of residues of interest.

5. The method of claim 4, wherein similar matching residues are residues which are determined to fall within a same predefined amino acid property group.

6. The method of any preceding claim, wherein the data indicative of the whole protein sequence corresponding to the first protein comprises a string of uppercase and lowercase characters, and wherein the lowercase characters are the annotations of the residues of interest.

7. The method of any preceding claim, wherein a profile hidden Markov model based algorithm is used to identify the whole protein sequences of the other proteins in the protein sequence database having the threshold level of sequence resemblance to the whole protein sequence of the first protein.

8. The method of any preceding claim, wherein an iterative refinement algorithm is used to perform the multiple sequence alignment on the whole protein sequences of the other proteins with respect to the whole protein sequence of the first protein.

9. The method of any preceding claim, wherein identifying the one or more of the other proteins as off-target proteins based on the measures of similarity comprises:
identifying the one or more of the other proteins as off-target proteins with respect the drug target in response to determining that each measure of similarity for the one or more of the other proteins exceeds a threshold measure of similarity.

10. A computer-readable medium comprising data or instruction code, which when executed on a processor, causes the processor to implement the computer-implemented method of any preceding claim.

11. A computer program product comprising instructions which, when the program is executed by a computer, cause the computer to carry out the method of any of claims 1 to 9.
